# EUROPEAN PATENT SPECIFICATION

(11) **EP 0 469 165 B1**
(45) Date of publication and mention of the grant of the patent: **13.03.1996**
(21) Application number: 90114624.1
(22) Date of filing: 30.07.1990
(51) Int. Cl.: A61F 2/12, A61B 19/00

(54) **Improved implant and inflating construction**
Implantat und Füllvorrichtung
Implant et dispositif de remplissage

(43) Date of publication of application: 05.02.1992
(73) Proprietor: MENTOR CORPORATION, Minneapolis, Minnesota 55411 (US)
(72) Inventor: Hilton, Becker, Palm Beach, Florida 33408 (US)
(74) Representative: Schwan, Gerhard, Dipl.-Ing.

(56) References cited:
- DE-B- 2 224 963
- FR-A- 2 199 266
- GB-A- 2 029 938
- US-A- 4 433 440
- US-A- 4 662 883
- US-A- 4 773 908

## Description

### BACKGROUND OF THE INVENTION

The present invention relates to implants, and more particularly to an inflatable implant including separate inner and outer lumens and an improved valve and filling tube construction as defined in claim 1. The present invention finds particular use in implants used in breast reconstruction and augmentation.

Breast reconstruction often is difficult after a mastectomy because of tight chest wall skin and scarring. It is desirable to expand the skin which allows the surgeon to avoid using skin from other parts of the body. To this end, temporary skin expanders have been used in breast reconstruction, and these are then replaced with a suitably sized, permanent implant. It will be appreciated that this requires two or more surgical procedures.

There are many permanent implants on the market which are useful in breast reconstruction and augmentation. The most commonly used is a single lumen, fixed volume implant utilizing silicone gel. The major drawback in this implant arises from gel bleed which causes capsular contracture. The major advantage is the natural feel which is extremely good.

In an attempt to overcome the "bleed" problem, saline filled implants have been developed. These have greatly reduced the capsule contracture problem, but the feel is not as good. Also, the saline filled implants are subject to spontaneous leakage caused by wave-like motion of the saline which is transmitted to the implant membrane.

There are various "combination" implants, some using a double lumen construction with gel in the inner chamber and saline in the outer chamber, and some with a polyurethane sponge coating to decrease capsule contracture.

A very important goal in implants of this nature is to achieve a natural feel and appearance and to minimize leakage and capsular contracture. Another goal is to facilitate expansion after implantation and to permit volume adjustment.

One implant currently available is that marketed by Cox-Uphoff International termed a Reverse Double Lumen Mammary (RDL_{TM}). This is a double membrane, double lumen construction having an outer membrane containing a gel and an inner membrane which is filled with saline. The inner and outer membranes are connected at a retention valve which allows insertion of a rigid filling tube from outside the implant into the inner lumen. By this construction, saline can be injected into the inner lumen at the time of implantation to fill the implant. Upon completion of the filling process and before skin closure, the filling tube is removed. For an explanation and understanding of the retention valve construction described here, reference may be made to US-A-4,178,643.

The double lumen construction of the CUI implant utilizing silicone gel in the outer lumen and saline in the inner provides a very natural and high quality appearance and feel. However, shear forces arise in this implant at the connected area of the membranes which increases the incidence of leakage. Also, the connected membranes inhibit free movement of the inner and outer membranes relative to each other and detracts from the natural feel and appearance of the implant.

In addition, the filling tube and filling valve constructions in those prior implants which can be filled at the time of surgery are not suitable for inflation or volume adjustment after implantation. A rigid filling tube is required and cannot be left in place for any long period of time because of the danger of puncturing the implant and because of discomfort to the patient caused by the rigid tube. Thus, these implants cannot function as an expander.

A further example of prior art implants is given in US-A-4 773 908.

It is important that the implant valves be constructed of a soft and pliable material because the valve is a part of the implant and remains in place with the implant. The filling tubes used with these implants often are in place in the valves for some time (in prior devices, before implantation) so that the soft and pliable valve material can become "set" and not recover its original shape when the tube is removed. Still further, such prior valves and filling tubes do not always sealingly cooperate in the desired fashion when the tube is in place. Thus, a relatively high incidence of leakage can result in these implants acting to the detriment of these devices as permanent implants.

### SUMMARY OF THE INVENTION

The present invention provides an implant having the desirable characteristics described above. This invention is a combination saline-gel prosthesis which provides a double membrane, double lumen implant wherein an outer membrane forming an outer lumen contains a viscous gel such as a silicone gel, and an inner membrane forming an inner lumen is adapted to be filled with saline for inflation of the implant. The membranes are totally separate from one another and the freedom of movement provided by this construction enhances and maximizes the natural feel and appearance of the implant.

Separate valves are provided in the inner and outer membranes, respectively, and are constructed so that a single, relatively soft and flexible filling tube connected to a reservoir can be used to percutaneously fill the inner lumen over an extended period of time and inflate the implant. The filling tube is sized to have an interference fit with the valves, and means is provided to reduce the cross-sectional dimension of the tube and to stiffen it during insertion. The valves are constructed so that the gel in the outer lumen sealingly cooperates with both valves and with the filling tube when the latter is in place enabling the filling tube to remain in place for an extended period of time. Once the implant is expanded to the desired volume, the filling tube is detached and the prosthesis remains in place as a permanent implant. Upon removal of the filling tube, the valves are prevented from leaking by the sealing action of the gel.

In addition to providing for tissue expansion, the implant allows for volume adjustment post-operatively while the filling tube and reservoir are still connected. Better symmetry of the breasts can be achieved if the implant volume can be adjusted several weeks after surgery when swelling has decreased and the implant has settled into position.

Furthermore, the implant of this invention facilitates a method of tissue expansion which eliminates the fear of pressure on the overlying skin and resulting necrosis from a large implant, so that the patient is reconstructed to the most appropriate breast size.

Additional objects and advantages of the invention will be set forth in part in the description which follows, and in part will be obvious from the description, or may be learned by practice of the invention. The objects and advantages of the invention may be realized and attained by means of the instrumentalities and combinations particularly pointed out in the appended claims.

To achieve the objects and in accordance with the purpose of the invention, as embodied and broadly described herein, the implant of this invention comprises inner and outer membranes, the outer membrane containing a viscous gel, first and second valves in the inner and outer membranes, respectively, and adapted to have a singular filling tube passed therethrough for inflating the implant, the gel in the outer membrane sealingly cooperating with the valves and with the filling tube when the latter is in place.

Preferably, the inner and outer membranes are separate and unattached from one another, as are the first and second valves, and the membranes are manually manipulated to align the valves for insertion of the singular filling tube. The inner lumen is filled preferably with saline to inflate the implant, and the valves each comprise separable flaps of soft, pliable material formed integral with the respective membranes and extending inwardly thereof and surrounding openings therein. The filling tube is soft and flexible and is insertable through the valve openings and operable to separate the flaps when passed therebetween, and the flaps are operable to close upon removal of the tube.

The filling tube may have a larger cross-sectional dimension than the connecting tubes, means for applying a longitudinal stretching force to the filling tube reducing its cross-sectional dimension to facilitate its insertion through the connecting tubes, the filling tube adapted to return to its original dimension upon removal of the stretching force, whereby to sealingly engage the connecting tube.

Preferably, the filling tube has a longitudinal passage therethrough which is closed at the distal end of the tube and which communicates with a transverse exit passage. The stretching force applying means includes an elongated rod adapted to be inserted through the passage to engage the closed end of the tube. The tube is removed from the valve by pulling which stretches the tube and reduces its cross-section.

The accompanying drawings, which are incorporated in and constitute a part of this specification, illustrate one embodiment of the invention and, together with the description, serve to explain the principles of the invention.

### BRIEF DESCRIPTION OF THE DRAWINGS

Fig. 1 is a perspective view of an implant constructed according to the present invention shown before inflation and with a filling tube in place;
Fig. 2 is a sectional view of the implant of Fig. 1 after inflation;
Fig. 3 is an enlarged sectional view of the implant of Figs. 1 and 2 and showing the filling tube in position for insertion;
Fig. 4 is a view similar to Fig. 3 and showing the filling tube inserted in the implant and longitudinally stretched;
Fig. 5 is a view similar to Fig. 4 and showing the filling tube stretching means removed and the filling tube relaxed; and
Fig. 6 is a view similar to Fig. 5 and showing the filling tube detached from the implant.

### DESCRIPTION OF THE PREFERRED EMBODIMENT

Reference will now be made in detail to the present preferred embodiment of the invention, an example of which is illustrated in the accompanying drawings.

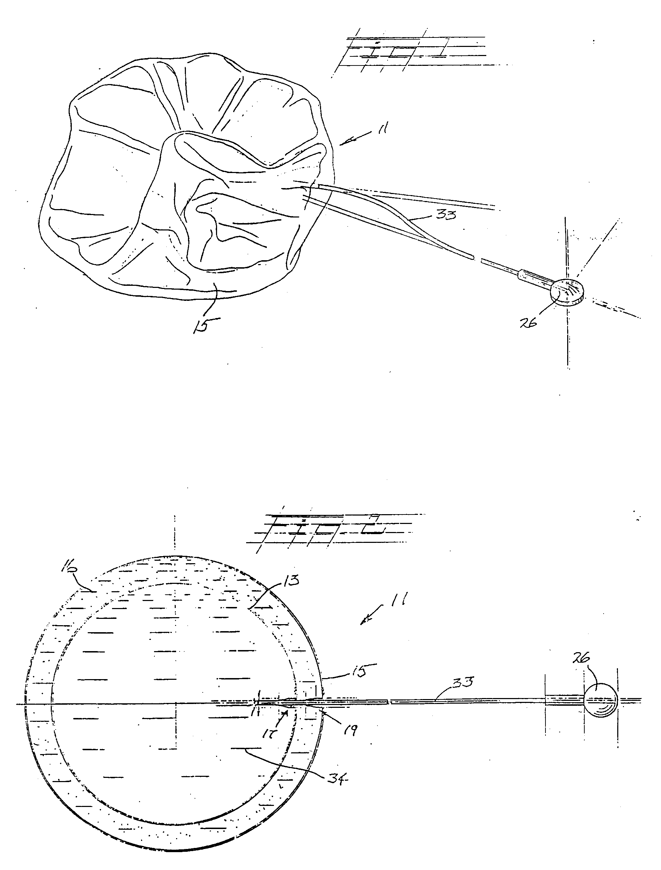

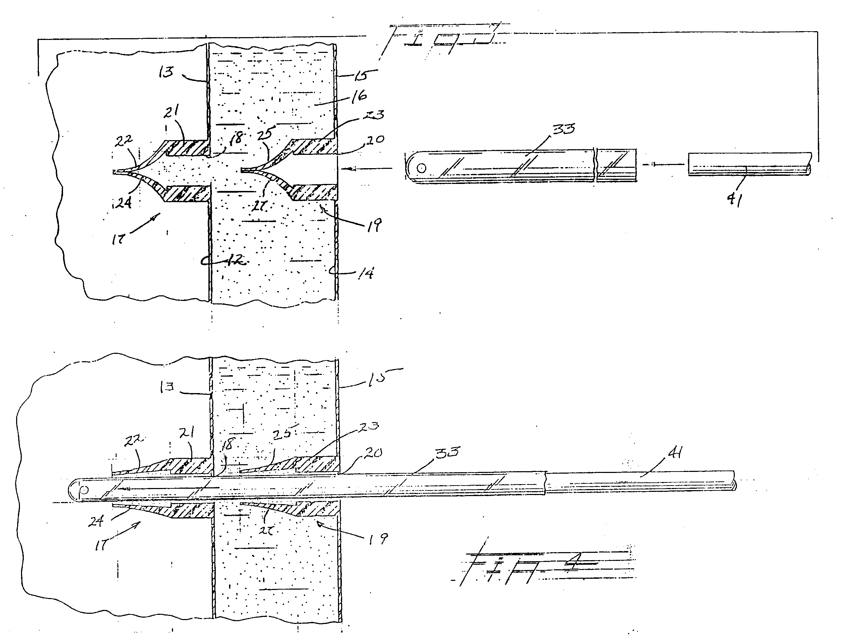

The preferred embodiment of the implant is shown in Figs. 1 and 2 and is represented generally by the numeral 11. The implant comprises inner and outer membranes, the outer membrane containing a viscous gel. As embodied herein, the implant 11 includes inner and outer membranes 13, 15 which are generally concentric to one another and form inner and outer concentric lumens 12, 14, respectively (see Fig. 2). The membranes 13, 15 are constructed of a suitable material such as a medical grade silicone rubber which does not react with human tissue, as will be understood by those skilled in the art. The outer membrane 15 contains an amount of viscous gel 16, for example, a silicone rubber gel of medical grade silicone, for purposes to be described.

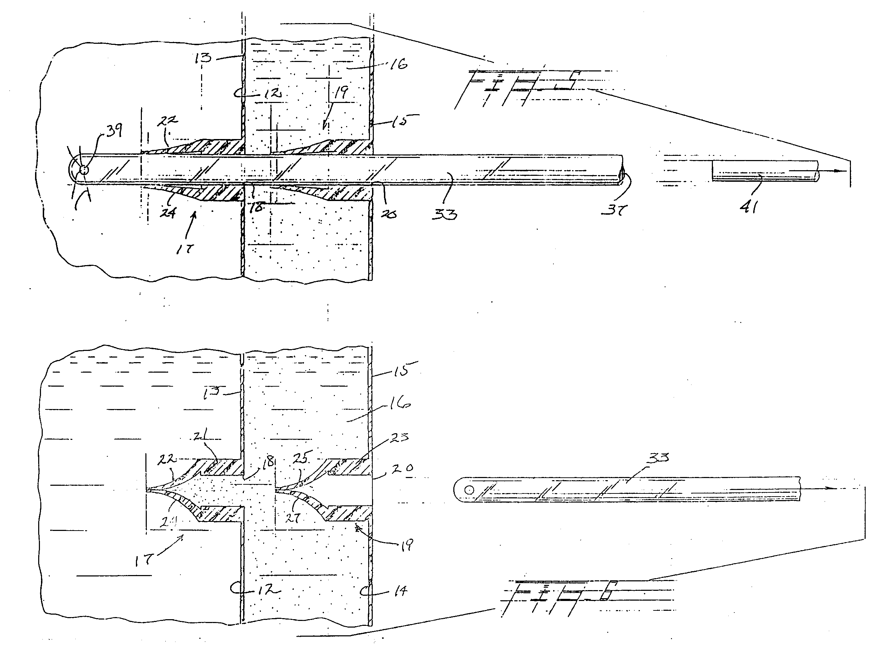

In accordance with the invention, first and second valves are provided in the inner and outer membranes, respectively. The valves are adapted to have a singular filling tube passed therethrough inflating the implant. As embodied herein and shown in Fig. 2, the inner and outer membranes 13, 15 are provided with valves 17, 19, respectively. The valves include short connecting tubes 21, 23 which surround openings 18, 20 in the membranes 13, 15, respectively, and are formed integral with the membranes and extend inwardly thereof. (See also Fig. 3). A pair of opposed flaps 22, 24 extend inwardly of and surround the tube 21 on the inner membrane 13. In substantially the same fashion, a pair of opposed flaps 25, 27 extend inwardly of and surround the tube 23 on the outer membrane 15.

In accordance with the invention and as further embodied herein, the membranes 13, 15 are separate from and unattached to one another as are the valves 17, 19. The membranes 13, 15 are manually manipulable to align the valves 17, 19 so that a singular filling tube can be passed through the valves for filling the inner membrane and expanding the implant.

A filling tube 33 is shown in place extending through the valves 17, 19 in Figs. 2 and 5. The filling tube 33 is relatively soft so as not to puncture either of the membranes, and can be inserted as shown at the time the implant 11 is manufactured. Alternatively, a filling tube can be inserted later particularly if one becomes damaged or if it accidentally becomes separated from the implant 11. In either case, the filling tube 33 extends through opening 20, tube 23, and flaps 25, 27 on outer membrane 15, and opening 18, tube 21, and flaps 22, 24 on inner membrane 13. The distal end of tube 33 is connected to a liquid source such as a reservoir 26 and used to fill the inner membrane with a liquid, such as saline 34 and expand the implant over an extended period of time. Upon completion of the filling (and expansion) process, the filling tube 33 is detached from the implant 11 and, in a manner described below, the valves 17, 19 close. For a description of an inflatable permanent implant having a detachable filling tube and reservoir, reference may be made to applicant's copending Patent US-A-4 643 733.

As described above, outer membrane 15 contains a silicone gel 16 such as a cohesive silicone rubber gel of medical grade silicone as is used in the Reverse Double Lumen Mammary (RDL_{TM}) currently marketed by Cox-Uphoff International of Santa Barbara, California. Although the quantity of gel in the outer lumen may vary according to the size of the implant, approximately 40-50 cu. cm. is preferred.

The inner membrane 13 is void prior to implantation of the implant. A small amount of saline (approximately 10% to 20% of its maximum recommended inflation amount) is inflated after implantation to expand the implant to the desired size by delivery of a corresponding quantity of saline 34 thereto. Saline is delivered to the inner membrane 13 by means of the filling tube 33 which, because of its relatively soft and flexible nature, can remain in place for a long period of time after implantation. This allows the implant to be expanded by percutaneous injections into the reservior over an extended period of time after surgery and provides for volume adjustment of the implant. Once the desired size is achieved, the filling tube 33 is detached. The entire interior contents of the expanded implant 11, i.e., the saline 34 and the gel 16, are under pressure so that flaps 22, 24 and 25, 27 are caused to close thereby sealing the valves 17, 19.

It will be appreciated that once the filling tube 33 is removed, the membranes 13, 15 are totally free to move relative to one another so that the implant 11 is free of any shear forces which otherwise would be present at a connection point between the membranes. This adds to the natural feel and appearance of the implant. The gel in the outer membrane 15 lubricates both membranes and provides the desirable characteristics of a natural breast formation including softness and suppleness. The saline-filled inner membrane 13 provides the necessary adjustment for the over-all size of the implant 11 and, in combination with the gel-filled outer membrane, provides the necessary and desirable round contour of the implant.

In accordance with the invention, the gel in the outer membrane sealingly cooperates with the valves and with the filling tube when the latter is in place. As embodied herein and shown in Figs. 2 and 5, the connecting tubes 21, 23 are sized to snugly receive the filling tube 33. The flaps 22, 24 and 25, 27 are formed and interconnected with tubes 21, 23 in a manner which produces a biasing force causing flaps 22, 24 to engage and flaps 25, 27 to engage when filling tube 33 is withdrawn.

In actual practice, the filling tube 33 may remain in place in the valves 17, 19 for a long period of time, sometimes for several weeks. In that case, the flaps 22, 24 and 25, 27 may become "set" so that upon removal of the filling tube 33, the flaps may not close fully in spite of the biasing force and the pressure assistance of the saline and gel. In that case, the gel 16 in the outer membrane 15 fills any gap between the flaps 25, 27 which are located in the gel containing outer lumen 14. With the filling tube 33 removed, gel 16 can flow into the short tube 21 on the inner membrane 13 to fill any gap between flaps 22, 24. Thus, the gel 16 effects a proper seal at both valves 15, 17.

As described above, the filling tube 33 preferably is relatively soft and flexible to minimize the likelihood of damage to or puncturing of the membranes 13, 15 and to prevent discomfort to the patient. The short connecting tubes 21, 23 preferably are somewhat stiffer than the filling tube 33. Desirably, a snug fit exists between the filling tube 33 and the connecting tubes 21, 23 and provides an effective seal therebetween as well as to help retain the tube 33 in place. Nevertheless, some crevices or gaps can exist between the filling tube 33 and the connecting tubes 21, 23 which can result in leakage past the valves 15, 17 while the filling tube 33 is in place.

Furthermore, the membranes 13, 15 and the filling tube 33 stretch to an extent as membrane 13 is filled with saline. This may cause some distortion of the connecting tubes 21, 23 which may add to or create crevices or spaces between the filling tube and the connecting tubes 21, 23.

In accordance with the invention and as embodied herein, gel 16 in the outer lumen coats the filling tube 33 during insertion and seeks out and fills any crevices or spaces between the filling tube 33 and the connecting tubes 21, 23 of valves 15, 17 when the filling tube 33 is in place. Thus, the gel 16 sealingly cooperates with the valves 15, 17 and with the filling tube and valves when the filling tube is in place.

In accordance with the invention, means is provided to reduce the cross-sectional dimension of the filling tube during insertion through the membrane valves, and to allow the filling tube to expand after insertion into snug engagement with the connecting tubes.

As embodied herein, the filling tube 33 is sized to provide an interference fit with the connecting tubes 21, 23. The filling tube 33 has an elongated passage 37 therethrough which is closed at the distal end of the tube and which communicates with a transverse exit passage 39. When the filling tube is in place in the implant 11, as shown in Fig. 5, saline from the reservoir 26 flows through passage 37 and exits passage 39 and enters the inner lumen 12.

To facilitate insertion of the soft, flexible filling tube 33 into the implant valves 15, 17, a rigid rod or wire 41 is inserted through the tube passage 37 and, by applying force against the closed end of tube 33, causes it to stretch longitudinally. This reduces the cross-sectional dimension of the filling tube 33, at the same time stiffening it, so that the filling tube 33 is easily inserted through the connecting tubes 21, 23 of valves 15, 17 (see Fig. 4). When insertion is complete, the wire or rod 41 is withdrawn and the filling tube 33 returns to its normal size and snugly engages the connecting tubes 21, 23 (Fig. 5).

When the filling tube 33 is to be detached, a simple pulling force is applied (Fig. 6). Gel which will have coated the filling tube 33 during insertion helps removal. Also, if the holding force of the connecting tubes 21, 23 resists removal of the filling tube, the pulling force on the tube causes it to stretch and its cross-sectional dimension to reduce, thereby facilitating removal.

In accordance with the invention, the implant valve and filling tube construction described above is useful in an implant comprising at least one membrane in which case a valve including a connecting tube is connected to the membrane and extends inwardly thereof. The connecting tube has self-sealing valve means at its inner end. The filling tube is constructed, inserted through, and detached from the valve as described above.

By the foregoing, there has been disclosed an improved implant, valve and filling tube construction calculated to fulfill the inventive objects set forth above and inherent herein. It will be apparent to those skilled in the art that various additions, substitutions, modifications and omissions can be made to the implant of the present invention. Thus, it is intended that the present invention cover the additions, substitutions, modifications and omissions provided they come within the scope of the appended claims.

## Claims

1. An implant, filling valve and filling tube construction comprising inner and outer membranes (13, 15), said outer membrane (15) containing a viscous gel (16), a valve (19) in said outer membrane extending inwardly of said outer membrane, and including a connecting tube (23) connected to the outer membrane, a second tube (21) in said inner membrane (13) and extending inwardly of said inner membrane, said valve (19) and said tubes (21, 23) being adapted to have a singular filling tube (33) passed therethrough for filling said inner membrane (13) and inflating said implant (11), said second tube (21) being seized to snugly engage the filling tube (33), and said gel (16) cooperating with said valve (19), said tube (21) and said filling tube (33) when the latter is in place, **characterized in that** said filling tube (33) is entirely removable from said implant (11) and that said inner membrane (13) is provided with a valve (17) including said second tube (21) connected to the inner membrane (13) and extending inwardly thereof, said connecting tube (23) being seized to snugly engage the filling tube (33), the valves (17,15) comprising self-sealing valve means including opposed flaps (22, 24; 25, 27) normally cooperatively engaging, said flaps being adapted to part upon passage of said filling tube (33) therebetween.

2. The implant and filling valve construction claimed in claim 1, said filling tube (33) being soft and flexible, whereby to allow said tube to remain in place without puncturing said implant (11).

3. The implant and filling valve construction claimed in claim 2, said filling tube (33) having a larger cross-sectional dimension than said connecting tubes (21, 23).

4. The implant and filling valve construction claimed in any one of the preceding claims, said inner membrane (13) being filled with saline (34).

5. The implant and filling valve construction claimed in any one of the preceding claims, said inner and outer membranes (13, 15) and said first and second valves (17, 19) being separate and unattached to one another, whereby said membranes are freely movable relative to one another.

6. The implant and filling valve construction claimed in any one of the preceding claims, having means (41) for applying a longitudinal stretching force to said filling tube (33) reducing its cross-sectional dimension to facilitate its insertion through said connecting tubes (21, 23) said filling tube adapted to return to its original dimension upon removal of the stretching force, whereby to sealingly engage said connecting tubes.

7. The implant and filling valve construction claimed in claim 6, said filling tube (33) having an elongated passage (37) therethrough which is closed at the distal end of said tube, said stretching force applying means including an elongated rod (41) adapted to be inserted through said passage to engage the closed end of said tube.

8. The implant and filling valve construction as claimed in claim 7, said elongated passage (37) communicated with a transverse exit passage (39).

## Patentansprüche

1. Implantat-, Füllventil- und Füllrohr-Konstruktion mit Innen- und Außermembranen (13, 15), wobei die Außenmembran (15) ein viskoses Gel (16) enthält, ein Ventil (19) in der Außenmembran sich von der Außenmembran nach innen erstreckt und ein mit der Außenmembran verbundenes Verbindungsrohr (23) aufweist, ein zweites Rohr (21) in der Innenmembran (13) sich von der Innenmembran nach innen erstreckt, durch das Ventil (19) und die Rohre (21, 23) ein einzelnes Füllrohr (33) zum Füllen der Innenmembran (13) und zum Aufweiten des Implantats (11) hindurchführbar ist, das zweite Rohr (21) bemessen ist, um mit dem Füllrohr (33) in engen Eingriff zu kommen, und das Gel (16) mit dem Ventil (19), dem Rohr (21) und dem Füllrohr (33) zusammenwirkt, wenn sich letzteres an Ort und Stelle befindet, dadurch gekennzeichnet, daß das Füllrohr (33) aus dem Implantat (11) vollständig herausnehmbar ist und daß die Innenmembran (13) mit einem Ventil (17) versehen ist, welches das mit der Innenmembran (13) verbundene und sich von dieser nach innen erstreckende zweite Rohr (21) aufweist, das Verbindungsrohr (23) bemessen ist, um mit dem Füllrohr (33) in engen Eingriff zu kommen, und die Ventile (17, 19) selbstdichtende Ventilanordnungen mit einander gegenüberliegenden Klappen (22, 24; 25, 27) aufweisen, die normalerweise zusammenwirkend in Eingriff miteinander stehen und die voneinander getrennt werden können, wenn das Füllrohr (33) zwischen ihnen hindurchtritt.

2. Implantat- und Füllventilkonstruktion nach Anspruch 1, bei welcher das Füllrohr (33) weich und flexibel ist, so daß das Rohr an Ort und Stelle verbleiben kann, ohne das Implantat (11) zu durchstoßen.

3. Implantat- und Füllventilkonstruktion nach Anspruch 2, bei der das Füllrohr (33) eine größere Querschnittsabmessung als die Verbindungsrohre (21, 23) hat.

4. Implantat- und Füllventilkonstruktion nach einem der vorhergehenden Ansprüche, bei der die Innenmembran (13) mit Salzlösung (34) gefüllt ist.

5. Implantat- und Füllventilkonstruktion nach einem der vorhergehenden Ansprüche, bei welcher die Innen- und Außenmembranen (13, 15) sowie das erste und das zweite Ventil (17, 19) voneinander getrennt und nicht miteinander verbunden sind, so daß die Membranen mit Bezug aufeinander frei bewegbar sind.

6. Implantat- und Füllventilkonstruktion nach einem der vorhergehenden Ansprüche mit einer Anordnung (41) zum Aufbringen einer Längsstreckkraft auf das Füllrohr (33), wodurch dessen Querschnittsabmessung vermindert wird, um das Einsetzen des Füllrohrs durch die Verbindungsrohre (21, 23) hindurch zu erleichtern, wobei das Füllrohr nach Beseitigen der Streckkraft zu seiner Ursprungsabmessung zurückkehren kann, um mit den Verbindungsrohren in Dichteingriff zu kommen.

7. Implantat- und Füllventilkonstruktion nach Anspruch 6, bei welcher das Füllrohr (33) mit einem langgestreckten Durchlaß (37) versehen ist, der an dem distalen Ende dieses Rohres geschlossen ist, wobei die Anordnung zum Aufbringen einer Streckkraft einen langgestreckten Stab (41) aufweist, der durch den Durchlaß hindurch einsetzbar ist, um mit dem geschlossenen Ende des Füllrohrs in Eingriff zu kommen.

8. Implantat- und Füllventilkonstruktion nach Anspruch 7, bei welcher der langgestreckte Durchlaß (37) mit einem quer verlaufenden Austrittsdurchlaß (39) in Verbindung steht.

## Revendications

1. Construction d'un implant, d'une valve de remplissage et d'un tube de remplissage comportant des membranes intérieure et extérieure (13, 15), ladite membrane extérieure (15) contenant un gel visqueux (16), une valve (19) dans ladite membrane extérieure s'étendant vers l'intérieur de ladite membrane extérieure et comprenant un tube (23) de raccordement raccordé à la membrane extérieure, un second tube (21) dans ladite membrane intérieure (13) et s'étendant vers l'intérieur de ladite membrane intérieure, ladite valve (19) et lesdits tubes (21, 23) étant conçus pour être traversée par un tue de remplissage unique (33) pour le remplissage de ladite membrane intérieure (13) et le gonflage dudit implant (11), ledit second tube (21) étant dimensionné pour s'ajuster étroitement au tube de remplissage (33), et ledit gel (16) coopérant avec ladite valve (19), ledit tube (21) et ledit tube de remplissage (33) lorsque ce dernier est en place, caractérisée en ce que ledit tube de remplissage (33) peut être enlevé librement dudit implant (11) et en ce que ladite membrane intérieure (13) est pourvue d'une valve (17) comprenant ledit second tube (21) raccordé à la membrane intérieure (13) et s'étendant vers l'intérieur de celle-ci, ledit tube de raccordement (23) étant dimensionné pour s'ajuster étroitement au tube de remplissage (33), les valves (17, 15) comportant des moyens à valves à auto-obturation comprenant des clapets opposés (22, 24 ; 25, 27) coopérant normalement en portant l'un contre l'autre, lesdits clapets étant conçus pour s'écarter lors du passage dudit tube de remplissage (33) entre eux.

2. Construction d'implant et de valve de remplissage selon la revendication 1, dans laquelle ledit tube de remplissage (33) est mou et flexible afin de permettre audit tube de rester en place sans perforer ledit implant (11).

3. Construction d' implant et de valve de remplissage selon la revendication 2, dans laquelle ledit tube de remplissage (33) présente en section transversale une dimension supérieure à celle desdits tubes de raccordement (21, 23).

4. Construction d'implant et de valve de remplissage selon l'une quelconque des revendications précédentes, dans laquelle ladite membrane intérieure (13) est remplie de solution saline (34).

5. Construction d'implant et de valve de remplissage selon l'une quelconque des revendications précédentes, dans laquelle lesdites membranes intérieure et extérieure (13, 15) et lesdites première et seconde valves (17, 19) sont séparées et ne sont pas reliées entre elles, grâce à quoi lesdites membranes peuvent se déplacer librement l'une par rapport à l'autre.

6. Construction d'implant et de valve de remplissage selon l'une quelconque des revendications précédentes, comportant des moyens (41) destinés à appliquer une force d'étirement longitudinale audit tube de remplissage (33), réduisant sa dimension en section transversale pour faciliter son insertion dans lesdits tubes de raccordement (21, 23), ledit tube de remplissage étant conçu pour revenir vers sa dimension d'origine lorsque la force d'étirement est relâchée, de façon à entrez en contact étanche avec lesdits tubes de raccordement.

7. Construction d' implant et de valve de remplissage selon la revendication 6, dans laquelle ledit tube de remplissage (33) est parcouru par un passage allongé (37) qui est fermé à l'extrémité distale dudit tube, lesdits moyens d'application d'une force d'étirage comprenant une tige allongée (41) destinée à être insérée dans ledit passage pour porter contre l'extrémité fermée dudit tube.

8. Construction d'implant et de valve de remplissage selon la revendication 7, dans laquelle ledit passage allongé (37) communique avec un passage transversal (39) de sortie.
